# EUROPÄISCHE PATENTSCHRIFT

(11) **EP 1 915 964 B1**
(45) Veröffentlichungstag und Bekanntmachung des Hinweises auf die Patenterteilung: **01.12.2010**
(21) Anmeldenummer: 07020688.3
(22) Anmeldetag: 23.10.2007
(51) Int. Cl.: A61B 19/00

(54) **Vorrichtung zur räumlichen Positionierung eines Gerätes**
Device for spatial positioning of an apparatus
Dispositif destiné au positionnement spatial d'un appareil

(30) Priorität: 27.10.2006 DE 102006050781
(43) Veröffentlichungstag der Anmeldung: 30.04.2008
(73) Patentinhaber: Ast GmbH, 07745 Jena (DE)
(72) Erfinder: Schwarze, Werner, Dr., 07743 Jena (DE)
(74) Vertreter: Engelhardt, Volker

(56) Entgegenhaltungen:
- EP-A- 1 649 818
- WO-A-2007/085953
- GB-A- 2 074 337
- US-A- 2 659 953
- US-A- 3 783 262
- US-A- 5 810 712
- US-A1- 2003 202 841

## Beschreibung

Die Erfindung bezieht sich auf eine Vorrichtung nach den Oberbegriffen der Patentansprüche 1 oder 2.

Aus der DE 3427 001 C1 ist eine solche Ortungs- und Positioniervorrichtung bekannt geworden, die im Wesentlichen entlang einer C-förmig gebogenen Schiene im Raum verfahrbar ist. Das Behandlungszentrum des Patienten wird vorzugsweise im Zentrum der C-förmig ausgebildeten Schiene angeordnet. eine solche Vorrichtung kann sowohl Röntgen- als auch um Stoßwellenerzeugungsgeräte zur Behandlung des Knochenwachstums bzw. zur Zertrümmerung von Nierensteinen abstützen. Die Ausrichtung des medizinischen Gerätes erfolgt demnach auf einem Kreisabschnitt, der in einer gemeinsamen Ebene mit dem Behandlungszentrum des Patienten liegt.

Als nachteilig bei diesem Stand der Technik hat sich herausgestellt, dass das Behandlungszentrum zentrisch in Bezug auf die kreisbogenförmig ausgestaltete Haltevorrichtung zu positionieren ist, um die Röntgenstrahlen bzw. den Fokuspunkt für die erzeugten Stoßwellen gerätespezifisch anzuordnen. Dies bedeutet, dass bereits geringe Positionierungenauigkeiten außerhalb des Mittelpunktes der Haltevorrichtung zu ungewünschten medizinischen Nebeneffekten führen. Beispielsweise weisen die Stoßwellen eine Charakteristik auf, die für den gewünschten Behandlungserfolg in dieser Position nicht geeignet sind. Daher ist permanent zu gewährleisten, dass das Behandlungszentrum des Patienten exakt im räumlichen Bereich des Zentrums der bogenförmigen Haltevorrichtung angeordnet ist.

Des Weiteren ist nachteilig, dass das Gerät ausschließlich in einer Ebene in Bezug auf das Behandlungszentrum ausgerichtet werden kann. Folglich sind die geometrischen Anpassungen, um das Behandlungszentrum im vorgegebenen Zentrumspunkt anzuordnen, zeitaufwendig vorzunehmen, da das Behandlungszentrum, also der Patient, in Bezug auf die Haltevorrichtung exakt zu positionieren ist und während der Behandlungsdauer in dieser Position fixiert werden muß. Eine Verfahrbarkeit des Gerätes entlang der Haltevorrichtung ist nämlich beim bekannten Stand der Technik ausschließlich entlang der C-förmig gebogenen Schiene möglich, so dass hierdurch eine zweidimensionale Ausrichtung des Gerätes vorliegt.

Aus der EP 1649818 A2 kann eine Vorrichtung zur Abstützung eines medizinischen Gerätes und dessen räumlichen Positionierung entnommen werden. In das Gerät ist eine runde Bohrung eingearbeitet, in die ein Stift oder Bolzen eingesteckt Ist, so dass ein Kugelgelenk entsteht, durch das das Gerät an einem Arm, der an einer Halteeinrichtung angebracht ist, verschweißt werden kann. Das Gerät ist folglich in einer Ebene ausrichtbar.

Als nachteilig hat sich herausgestellt, dass aufgrund der konstruktiven Ausgestaltung des Gelenkes lediglich ein Verschwenkwinkel um eine Achse vorhanden ist, dessen Verschwenkbereich zudem begrenzt ist. Das medizinische Gerät ist nämlich über den Stift oder Bolzen, an dem Arm der Halteeinrichtung abgestützt und lediglich um die Achse des Bolzens kann das Gerät in einer Ebene verschwenkt werden.

Es ist daher Aufgabe der Erfindung, eine Vorrichtung der eingangs genannten Gattung bereitzustellen, mittels der das Gerät im Raum in Bezug auf ein Behandlungszentrum eines Patienten ausrichtbar und feststellbar ist. Die Bewegung des Gerätes soll einfach und effizient sein.

Diese Aufgabe wird dadurch gelöst, dass das Gerät in einem Verstellwinkel (β) in zwei unterschiedlichen Verstell-Abschnitten aufgeteilt ist.

Weitere vorteilhafte Weiterbildungen der Erfindung ergeben sich aus den Unteransprüchen.

Es ist besonders vorteilhaft, wenn die Innenseite des Aufnahmekörpers beabstandet zu der Außenmantelfläche angeordnet ist, wenn die Innenseite konzentrisch zu der Außenmantelfläche verläuft und wenn zwischen dem Aufnahmekörper und an diesem abgestützt mindestens ein Gleit- oder ein Wälzlager vorgesehen ist, das auf die Außenmantelfläche einwirkt, da durch das vorhandene Gleit- oder Wälzlager eine Relativbewegung zwischen dem Gerät und dem Aufnahmekörper als Bestandteil der Haltevorrichtung möglich ist und diese Relativbewegung in Richtung von zwei der drei Raumvektoren durchführbar ist.

Die Bewegung zwischen der Außenmantelfläche und dem Aufnahmekörper ist durch die geometrische Ausgestaltung der Außenmantelfläche und die daran angepasste Innenkontur des Aufnahmekörpers gewährleistet, ohne dass das Gerät aus dem Aufnahmekörper aufgrund der Positioniermöglichkeiten verrutscht. Vielmehr umgreift der ringförmige Aufnahmekörper die Außenmantelfläche teilweise oder ganz, um diese abzustützen und gleichzeitig eine Relativbewegung im Raum zuzulassen.

Die Ausrichtung- und Positionierungsmöglichkeiten des Gerätes im Raum werden dadurch erhöht, dass die Haltevorrichtung aus einem Arm besteht, an dessen Ende ein Drehgelenk zwischen diesem und dem Aufnahmekörper vorgesehen ist, so dass das Gerät um die Längsachse des Armes verschwenkbar ist. Somit überlagern sich zwei Teilabschnitte für die Verstellungsmöglichkeit des Gerätes in einer vertikalen Ebene. Beide Teilabschnitte des Verstellwinkels sind räumlich begrenzt überlagernd sich, um einen gesamten Verstellwinkelbereich von +/- 45" zu erreichen. Des Weiteren kann das Gerät in einer Verstellebene, die senkrecht zu der Längsachse des Gerätes und parallel zu der Längsachse des Armes verläuft, in einem Verstellwinkel von +/- 26° bewegt werden, so dass durch eine Überlagerung der beiden Verstellbewegungen eine diagonale räumliche Ausrichtung des Gerätes insgesamt möglich ist.

Die Begrenzung des Verstellwinkels wird durch die geometrischen Abmessungen der Außenmantelfläche in Bezug auf den Durchmesser des zylinderförmig ausgebildeten Gerätes vorgegeben. Die zu der Außenmantelfläche unmittelbar benachbart angeordnete zylindrische Mantelfläche des Gerätes wirkt nämlich als Anschlag und liegt bei der maximal möglich eingestellten Auslenkung an dem Seitenbereich des Aufnahmekörpers an.

In der Zeichnung ist ein erfindungsgemäßes Ausführungsbeispiel dargestellt, das nachfolgend näher erläutert wird. Im Einzelnen zeigt:
- Figur 1: eine Vorrichtung zur räumlichen Positionierung eines Stoßwellenerzeugungs-Gerätes, das mittels eines Armes an einer Halteeinrichtung verschwenkbar abgestützt ist, in perspektivischer Ansicht,
- Figur 2: die Vorrichtung gemäß Figur 1, entlang der Schnittlinie II - II,
- Figur 3: die Vorrichtung gemäß Figur 2 entlang der Schnittlinie III - III, und
- Figur 4: eine vergrößerte Teilansicht der Vorrichtung aus Figur 2 im Bereich IV.

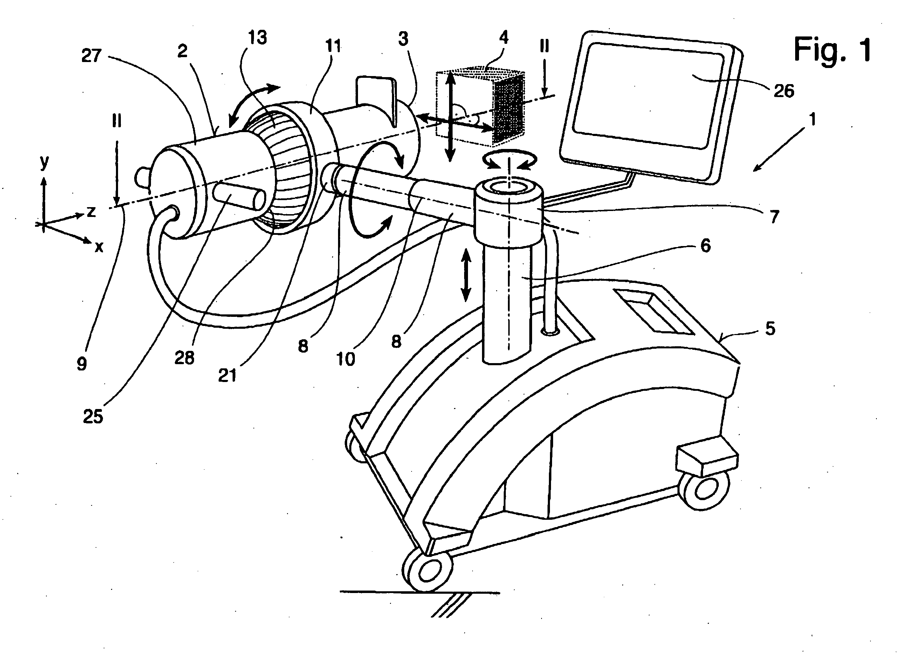

In Figur 1 ist eine Vorrichtung 1 dargestellt, durch die ein medizinisches Gerät 2, durch das beispielsweise Stoßwellen zur Zertrümmerung von Nierensteinen oder zur Bildung von Knochenwachstum oder zur Wundheilung erzeugt werden können. Die von dem Gerät 2 generierten Stoßwellen sollen durch ein Membranbalg 3 unmittelbar in ein schematisch dargestelltes Behandlungszentrum 4 gelangen, das im Inneren eines nicht dargestellten Patienten liegt. Der Membranbalg 3 berührt die Haut des Patienten oder ist von diesem beabstandet angeordnet. Das medizinische Gerät 2 kann auch als Röntgengerät oder dgl. ausgebildet und von dem Behandlungszentrum 4 oder dem Patienten beabstandet angeordnet sein.

Das Gerät 2 ist über einen L-förmig ausgebildeten Arm 6 an einer als Wagen ausgestalteten Halteeinrichtung 5 angebracht. Die Halteeinrichtung 5 ist demnach entlang eines Untergrundes verfahrbar. Der L-förmig ausgebildete Arm 6 besteht aus einem zwischen den Schenkeln des Armes 6 angeordneten Drehgelenk 7, so dass der Arm 6 um 360° in Bezug auf die Halteeinrichtung 5 verschwenkbar ist. Darüber hinaus kann das Drehgelenk 7 entlang des vertikalen Schenkels des Armes 6 höhenverstellbar verfahren werden.

Am freien Ende des Armes 6 ist ein Drehgelenk 21 vorgesehen, das nachfolgend noch näher erläutert wird. An dem Drehgelenk 21 ist ein Aufnahmekörper 11 angebracht, der in seiner Gesamtheit einen vollständig geschlossenen Ring bildet. Zu Montage- bzw. Demontagezwecken ist der Aufnahmekörper 11 zweiteilig ausgebildet. Ferner kann der Aufnahmekörper 11 als offener Ring ausgestaltet sein, der jedoch mindestens einen Ringabschnitt von 185° aufweist.

Das Gerät 2 besteht im Wesentlichen aus einem Gehäuse, das eine zylinderförmige Mantelfläche 27 aufweist. Etwa im geometrischen Zentrum 20 des Gerätes 2 ist eine kugelkalottenförmige Außenmantelfläche 13 vorgesehen, die vollständig oder teilweise von dem Aufnahmekörper 11 umgriffen ist. Durch den Aufnahmekörper 11 wird demnach das Gerät 2 an der Halteeinrichtung 5 derart abgestützt, dass das Gerät 2 relativ zu dem Aufnahmekörper 11 beweglich ist.

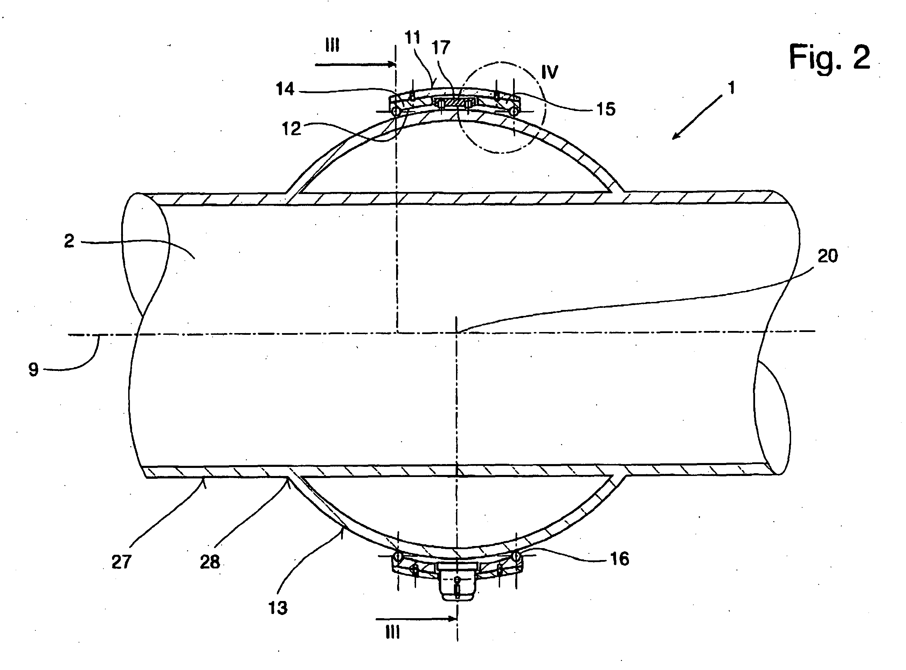

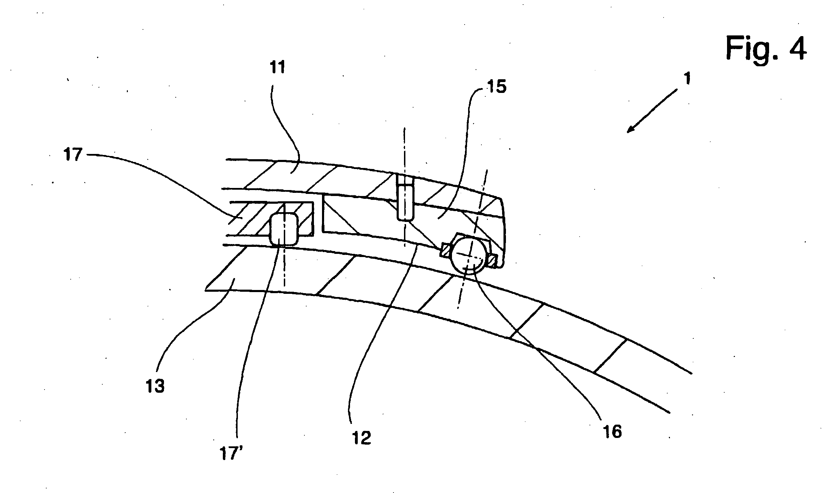

Den Figuren 2 und 4 kann entnommen werden, dass der Aufnahmekörper 11 eine konzentrisch gekrümmte Innenseite 12 aufweist, die zu der Außenmantelfläche 13 beabstandet verläuft. Zwischen diesen beiden Flächen soll ein konstanter Abstand vorliegen, um ein Gleit- oder Wälzlager unterzubringen, durch das das Gerät 2 verschwenkbar am Aufnahmekörper 11 abgestützt ist.

An der Innenseite 12 des Aufnahmekörpers 11 sind zwei Ringe 14 und 15 eingesetzt, in denen wiederum eine Vielzahl von Kugeln 16 drehbar angeordnet sind. Die Ringe 14 und 15 bilden demnach zusammen mit den Kugeln 16 zwei zueinander beabstandete Wälzlager, durch die die Außenmantelfläche 13 verschwenkbar gehalten ist, ohne dass das Gehäuse 2 aus dem Aufnahmekörper 11 in Richtung dessen Längsachse 9 herausgezogen werden kann. In ähnlich konstruktiver Weise können ein oder zwei Gleitlager im Inneren des Aufnahmekörpers 11 untergebracht sein. Die Längsachse 9 entspricht dabei der Z-Achse des in Figur 1 dargestellten Koordinatensystems; durch die von der x-und der y-Achse gebildeten Ebene wird die Bewegungsmöglichkeit des Gerätes 2 bzw. des Membranbalges 3 definiert.

Der Abstand zwischen der Innenseite 12 und der Außenmantelfläche 13 entspricht dem Kugelradius der Kugeln 16. Des Weiteren liegen die Kugeln 16 senkrecht auf der Außenmantelfläche 13 an, und zwar unabhängig von der Winkelstellung der Außenmantelfläche 13 in Bezug auf den Aufnahmekörper 11.

Des Weiteren ist der Innenseite 12 des Aufnahmekörpers 11 eine Bremse 17 zugeordnet, die in dem Aufnahmekörper 11 ortsfest gehalten ist und etwa mittig zwischen den beiden Ringen 14 und 15 verläuft. Die Bremse 17 wirkt auf die Außenmantelfläche 13 derart ein, dass die Bewegung des Gerätes 2 durch in die Bremse 17 eingesetzte Bremsglieder 17 festgestellt wird. Zu diesem Zweck ist die Bremse 17 aus zwei Halteringen zusammengesetzt, deren freie gegenüberliegende Enden durch ein Stellglied 18 zusammengehalten sind.

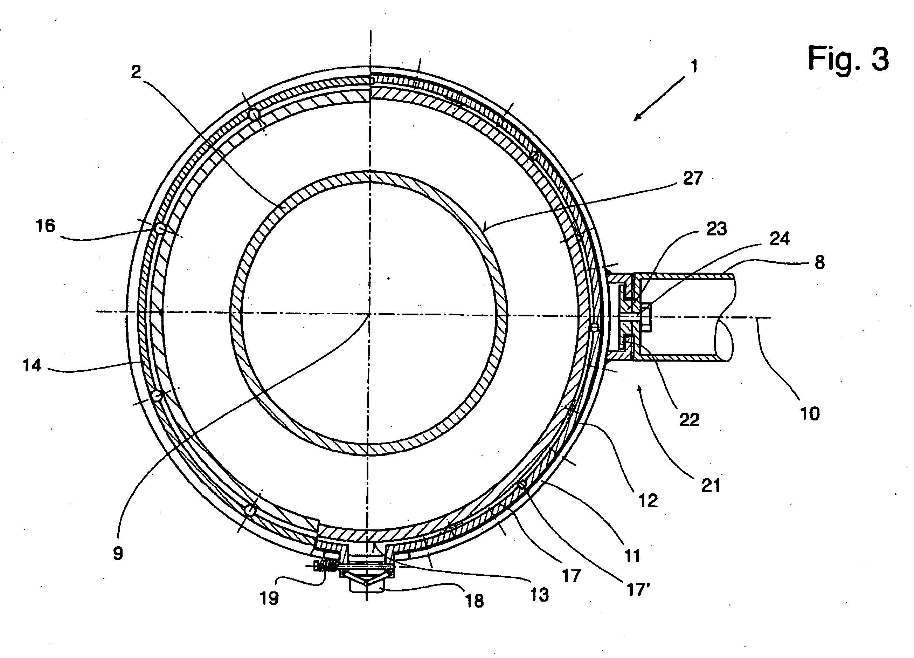

In Figur 3 ist die Funktionsweise des Stellgliedes 18 dargestellt, das über einen Winkelhebel mit den beiden freien Enden der Bremse 17 verbunden ist. Durch Bewegen des Stellgliedes 18 in Richtung des Gerätes 2 werden somit die beiden freien Ende aufgrund des vorhandenen Winkelhebels auseinandergedrückt und folglich vergrößert sich der Innendurchmesser der Bremse 17, wodurch die Reibkraft auf die Außenmantelfläche 13 reduziert wird bzw, gänzlich entfällt. Die Bremsglieder 17' sind folglich von der Außenmantelfläche 13 beabstandet angeordnet. Damit kann das Gerät 2 relativ zu dem Aufnahmekörper 11 bewegt werden. Durch Bewegen des Stellgliedes 18 wird des Weiteren eine Rückstellfeder 19 zusammengedrückt. Wird nunmehr das Stellglied 18 losgelassen, drückt die Rückstellfeder 19 die beiden freien Enden der Bremse 17 zusammen, so dass erneut eine Bremskraft durch die Bremsglieder 17' auf die Außenmantelfläche 13 einwirkt und daher eine Bewegung des Gerätes 2 relativ zu dem Aufnahmekörper 11 nicht oder nur mit erheblichem Kraftaufwand möglich ist.

Des Weiteren ist der Figur 3 zu entnehmen, dass der Aufnahmekörper 11 über das Drehgelenk 21, das in Form einer Schwalbenschwanzführung 22 ausgebildet ist, verbunden ist. Der eine Schenkel des Armes 6 ist dabei aus zwei teleskopartig ineinander verschieblichen Streben 8 aufgebaut. Zu Montagezwecken können die Streben 8 voneinander gelöst werden, so dass das Innere des dem Aufnahmekörper 11 zugeordneten Teilbereichs von außen zugänglich ist. Zur Abdeckung der Schwalbenschwanzführung 22 ist ein Deckel 23 vorgesehen, der durch eine im Inneren der Strebe 8 angeordnete Schraube 24 arretierbar ist. Somit kann der Aufnahmekörper 11 senkrecht um die Längsachse der Strebe 8 aufgrund des vorhandenen Drehgelenkes 21 bewegt werden.

Insbesondere der Figur 1 kann entnommen werden, in welchen Richtungen das Gerät 2 räumlich ausrichtbar ist. Zur manuellen Bedienbarkeit sind dabei an dem Gerät 2 zwei gegenüberliegende Haltegriffe 25 vorgesehen.

Das Gerät 2 kann um das Drehgelenk 21, also um die Längsachse der Streben 8, in vertikaler Richtung bewegt werden. Des Weiteren ist das Gerät 2 in einer Ebene, die parallel zu der Längsachse der Streben 8 verläuft, ausrichtbar, denn die Außenmantelfläche 13 kann innerhalb des Aufnahmekörpers 11 bewegt werden, und zwar so lange, bis die Zylindermantelfläche 27, die im Bereich der Außenmantelfläche 13 verläuft, als Anschlag 28 an der Stirnseite des Aufnahmekörpers 11 anliegt.

Im gewählten Ausführungsbeispiel sind die geometrischen Verhältnisse zwischen dem Durchmesser der Außenmantelfläche 13 und dem Innendurchmesser des Aufnahmekörpers 11 derart gewählt, dass ein Verstellwinkel α von +/- 26° um das Zentrum 20 des Gerätes 2 möglich ist. Hierbei handelt es sich um einen ersten Teilabschnitt des Verstellwinkels α. Ein zweiter Teilabschnitt des Verstellwinkels α wird über die Drehmöglichkeiten des Drehgelenkes 21 generiert. Die Auslenkung in vertikaler Richtung, also in der Ebene, die senkrecht zu der Längsachse 9 des Gerätes 2 verläuft, beträgt etwa +/- 45°.

Aufgrund des konstruktiven Aufbaus der Vorrichtung 1 ist es nunmehr möglich, dass der Patient bzw. dessen Behandlungszentrum 4 ortsfest und stationär verbleibt. Das Gerät 2 kann ohne dass das Behandlungszentrum 4 zu bewegen ist, exakt zu diesem positioniert werden. Hierfür ist ein Bildschirm 26 mit einem nicht dargestellten Ortungs- und Navigationssystem vorgesehen, durch das der behandelnde Arzt die Möglichkeit erhält, die Ausrichtung des Gerätes 2 in Bezug auf das Behandlungszentrum 4 permanent zu überwachen.

## Patentansprüche

1. Vorrichtung (1) zur räumlichen Positionierung eines medizinisch einsetzbaren Gerätes (2), umfassend einen Arm (6) sowie eine Haltevorrichtung (5), welche derart gestattet sind, dass das medizinische Gerät (2) mittels des Armes (6) an der Halteeinrichtung (5) verschwenkbar abstützbar ist, wobei an einem freien Ende des Armes (6) ein ringförmiger Aufnahmekörper (11) ausgebildet ist, derart gestaltet, dass von ihm eine kugelkalottenförmig gestaltete Außenmantelfläche (13) des Gerätes (2) ganz oder teilweise umgreifbar ist, wobei die Außenmantelfläche (13) des Gerätes (2) relativ zu dem Aufnahmekörper (11) verschwenkbar in diesem gehalten ist,
**dadurch gekennzeichnet,**
**dass** das Gerät (2) in einer ersten Ebene, die senkrecht zu der Längsachse (9) des Gerätes (2) verläuft, in einem Verstellwinkel (α) von +/- 26° und in einer zweiten Ebene, die senkrecht zu der ersten Ebene verläuft, in seinem Verstellwinkel (β) von +/- 45° beweglich ist.

2. Vorrichtung (1) nach Patentanspruch 1,
**dadurch gekennzeichnet,**
**dass** an dem freien Ende des Armes (6) ein ringförmig ausgebildeter Aufnahmekörper (11) angebracht ist, dass an dem Gerät (2) eine kugelkalottenförmig gestaltete Außenmantelfläche (13) vorgesehen ist, die von dem Aufnahmekörper (11) ganz oder teilweise umgriffen ist und dass die Außenmantelfläche (13) des Gerätes (2) relativ zu dem Aufnahmekörper (11) verschwenkbar in diesem gehalten ist,

3. Vorrichtung nach Anspruch 1 oder 2,
**dadurch gekennzeichnet,**
**dass** die Innenseite (12) des Aufnahmekörpers (11) beabstandet zu der Außenmantelfläche (13) angeordnet ist, dass die Innenseite (12) konzentrisch zu der Außenmantelfläche (13) verläuft und dass zwischen dem Aufnahmekörper (11) und an diesem abgestützt mindestens ein Gleit- oder ein Wälzlager (13, 15, 16) vorgesehen ist, das auf die Außenmantelfläche (13) einwirkt.

4. Vorrichtung nach Anspruch 3,
**dadurch gekennzeichnet,**
**dass** das Wälzlager aus zwei Ringen (14, 15) besteht, die im Inneren des Aufnahmekörpers (11) fest angebracht und beabstandet zueinander ausgerichtet sind, dass die zwei Ringe (14, 15) parallel zueinander und senkrecht zu der Längsachse des Gerätes (2) verlaufen, dass in den beiden Ringen (14, 15) jeweils eine Vielzahl von aus diesen abstehenden Rollkörpern, die vorzugsweise in Form von Kugeln (16) ausgestattet sind, eingesetzt sind, die auf der Außenmantelfläche (13) des Gerätes (2) aufliegen, und dass die Rollkörper (16) drehbar in dem jeweiligen Ring (14, 15) gelagert sind.

5. Vorrichtung nach Anspruch 4,
**dadurch gekennzeichnet,**
**dass** die beiden Ringe (14, 15) im Bereich der beiden Stirnseiten des Aufnahmekörpers (11) angeordnet sind, und dass die Krümmung der Ringe (14, 15) konzentrisch zu der Außenmantelfläche (13) verläuft.

6. Vorrichtung nach Anspruch 5,
**dadurch gekennzeichnet,**
**dass** jeder der Rollkörper (16) senkrecht auf der Außenmantelfläche (13) des Gerätes (2) aufliegt.

7. Vorrichtung nach einem oder mehreren der vorgenannten Ansprüche,
**dadurch gekennzeichnet,**
**dass** zwischen dem Aufnahmekörper (11) und dem Arm (6) ein Drehgelenk (21) angeordnet ist, durch das das Gerät (2) senkrecht zu der von dem Arm (6) gebildeten Achse (10) beweglich ausrichtbar ist.

8. Vorrichtung nach Anspruch 7,
**dadurch gekennzeichnet,**
**dass** das Drehgelenk (21) aus zwei drehbar ineinander gelagerten Führungsgliedern (22, 23) besteht, die in Form einer Schwalbenschwanzführung (22) miteinander verbunden sind.

9. Vorrichtung nach Anspruch 8,
**dadurch gekennzeichnet,**
**dass** die Öffnungen in der Schwalbenschwanzführung (22) durch einen Deckel (23) oder dgl. in Richtung der Längsachse (10) des Armes (6) verschlossen sind.

10. Vorrichtung nach einem oder mehreren der vorgenannten Ansprüche,
**dadurch gekennzeichnet,**
**dass** im Inneren des Aufnahmekörpers (11) eine von außen bedienbare Bremse (17) vorgesehen ist, die auf der Außenmantelfläche (13) anliegt und auf diese einstellbar einwirkt.

11. Vorrichtung nach Anspruch 10,
**dadurch gekennzeichnet,**
**dass** die Bremse (17) aus einem zweiteiligen Ring besteht, der die Außenmantelfläche (13) vollständig umgreift und dessen beiden freien Enden von außen mittels eines Stellgliedes (18) auseinander drückbar sind und dass die beiden freien Enden der Bremse (17) mittels einer Rückstellfeder (19) zusammengepresst sind.

12. Vorrichtung nach Anspruch 10 oder 11,
**dadurch gekennzeichnet,**
**dass** die Bremse (17) in der Mittelebne des Aufnahmekörper (11) angeordnet ist.

13. Vorrichtung nach einem oder mehreren der vorgenannten Ansprüche,
**dadurch gekennzeichnet,**
**dass** der Arm (6) aus zwei teleskopartig ineinander verschiebbaren Stützstreben (8) gebildet ist.

14. Vorrichtung nach einem der vorgenannten Ansprüche,
**dadurch gekennzeichnet,**
**dass** zur manuellen Ausrichtung des Gerätes (2) an diesem ein oder mehrere Haltegriffe (25) vorgesehen ist bzw. sind.

15. Vorrichtung nach einem der vorgenannten Ansprüche,
**dadurch gekennzeichnet,**
**dass** die Oberfläche des Gerätes (2), die parallel zu dessen Längsachse (9) und im Bereich der Außenmantelfläche (13) verläuft, als Anschlag (28) für die Bewegungsfreiheit des Gerätes (2) dient.

16. Vorrichtung nach Anspruch 1 oder 2,
**dadurch gekennzeichnet,**
**dass** der Verstellwinkel (β) in zwei unterschiedlichen Verstell-Abschnitten aufgeteilt ist.

## Claims

1. A device (1) for spatial positioning of an apparatus (2) with a medical application, comprising an arm (6) as well as a holding device (5), which are configured in such a way that the medical apparatus (2) is supported in a pivoting arrangement on the holding device (5) by means of the arm (6), with a ring-shaped holding body (11) attached to a free end of the arm (6), configured in such a way that a spherical cup-shaped outer jacket surface (13) is provided on the apparatus (2) and the surface can be enclosed in whole or in part by the holding body (11), with the outer jacket surface (13) of the apparatus (2) is held in the holding body (11) and can be swivelled relative to the holding body.
**characterised in that,**
the apparatus (2) can be moved in a first plane that runs perpendicular to the lengthways axis (9) of the apparatus (2) within an adjusting angle (α) of +/-26° and in a second plane that runs perpendicular to the first plane within an adjusting angle (β) of +/-45°.

2. The device (1) in accordance with Claim 1,
**characterised in that,**
a ring-shaped holding body (11) is attached to the free end of the arm (6), that a spherical cup-shaped outer jacket surface (13) is provided on the apparatus (2) with the surface enclosed wholly or in part by the holding body (11) and that the outer jacket surface (13) of the apparatus (2) is held in the holding body (11) and can be pivoted relative to the holding body (11).

3. The device in accordance with Claim 1 or 2,
**characterised in that,**
the inside (12) of the holding body (11) is arranged at a distance from the outer jacket surface (13), that the inside (12) runs concentrically to the outer jacket surface (13) and that at least one plain or anti-friction bearing (13, 15, 16) is provided between the holding body (11) and supported against it, with the bearing acting against the outer jacket surface (13).

4. The device in accordance with Claim 3,
**characterised in that,**
the anti-friction bearing consists of two rings (14, 15) that are permanently attached inside the holding body (11) and aligned with spaces in between them, that the two rings (14, 15) run parallel to one another and perpendicular to the lengthways axis of the apparatus (2), that a plurality of roller bodies are inserted in both rings (14, 15), project from the rings, are preferably shaped as balls (16) and are in contact with the outer jacket surface (13) of the apparatus (2), and that the roller bodies (16) are mounted in a rotating arrangement in the ring (14, 15) in question.

5. The device in accordance Claim 4,
**characterised in that,**
both rings (14, 15) are arranged in the area of both ends of the holding body (11) and that the curvature of the rings (14, 15) runs concentrically to the outer jacket surface (13).

6. The device in accordance with Claim 5,
**characterised in that,**
each of the roller bodies (16) contacts the outer jacket surface (13) of the apparatus (2) at a perpendicular angle.

7. The device in accordance with one or more of the aforementioned claims,
**characterised in that,**
a swivel joint (21) is arranged between the holding body (11) and the arm (6) by means of which the apparatus (2) can be movably aligned perpendicular to the axis (10) formed by the arm (6).

8. The device in accordance with Claim 7,
**characterised in that,**
the swivel joint (21) consists of two guide elements (22, 23) rotatably arranged one inside the other, which are preferably connected together in the form of a dovetail guide (22).

9. The device in accordance with Claim 8,
**characterised in that,**
the openings in the dovetail guide (22) are closed by a cover (23) or the like in the direction of the lengthways axis (10) of the arm (6).

10. The device in accordance with one or more of the aforementioned claims,
**characterised in that,**
an eternally operated brake (17) is provided in the inside of the holding body (11) and the brake contacts the outer jacket surface (13) and has an adjustable effect on it.

11. The device in accordance with Claim 10,
**characterised in that**,
the brake (17) consists of a two-part ring that completely encloses the outer jacket surface (13) and the two free ends of which can be pressed apart from the outside by means of an actuator (18), and that the two free ends of the brake (17) are pressed together by a return spring (19).

12. The device in accordance with Claim 10 or 11,
**characterised in that,**
the brake (17) is in the central plane of the holding body (11).

13. The device in accordance with one or more of the aforementioned claims,
**characterised in that,**
that the arm (6) is formed by two support struts (8) that can be telescoped one inside the other.

14. The device in accordance with one or more of the aforementioned claims,
**characterised in that,**
in order to align the apparatus (2) manually, one or more handles (25) is/are provided on the device.

15. The device in accordance with one or more of the aforementioned claims,
**characterised in that,**
the surface of the apparatus (2) that runs parallel to the lengthways axis (9) of the device and in the area of the outer jacket surface (13) serves as the stop (28) for the freedom of movement of the apparatus (2).

16. The device in accordance with Claim 1 or 2,
**characterised in that,**
the adjusting angle (β) is divided into two different adjustment sections.

## Revendications

1. Dispositif (1) destiné au positionnement spatial d'un appareil (2) utilisé à des fins médicales, comprenant un bras (6) et un dispositif de retient (5), conçu de sorte que moyennant le bras (6), l'appareil médical (2) s'appuie pivotant sur le dispositif de retient (5), une extrémité libre du bras (6) comportant un corps récepteur annulaire (11) qui est formé de sorte qu'il saisisse partiellement ou complètement une enveloppe extérieure (13) de l'appareil (2) ayant la forme d'une calotte sphérique et où l'enveloppe extérieure (13) de l'appareil (2) est retenue dans le corps récepteur annulaire (11) de manière à se laisser pivoter relativement à celui,
**caractérisé en ce que**
l'appareil (2) se laisse mouvoir dans un angle de réglage (α) de ± 26°dans un premier plan orienté perpendiculairement à l'axe longitudinal (9) de l'appareil (2), et dans un angle de réglage (β) de ± 45° dans un second plan orienté perpendiculairement au premier plan.

2. Dispositif (1) d'après la revendication 1,
**caractérisé en ce que**
sur l'extrémité libre du bras (6), il est prévu un corps récepteur annulaire (11), que sur l'appareil (2), il est prévu une enveloppe extérieure (13) sous la forme d'une calotte sphérique, qui est saisie partiellement ou complètement par le corps récepteur (11), et que l'enveloppe extérieure (13) de l'appareil (2) est retenue dans le corps récepteur annulaire (11) de manière à se laisser pivoter relativement à celui.

3. Dispositif d'après une des revendications 1 ou 2,
**caractérisé en ce que**
la face intérieure (12) du corps récepteur (11) est disposée à une certaine distance de l'enveloppe extérieure (13), que la face intérieure (12) est concentrique par rapport à l'enveloppe extérieure (13) et que sur le corps récepteur (11), il s'appuie au moins un palier à glissement ou un roulement à rouleaux (13, 15, 16) agissant sur l'enveloppe extérieure (13).

4. Dispositif d'après la revendication 3,
**caractérisé en ce que**
le roulement à rouleaux consiste de deux anneaux (14, 15) fixés rigidement à l'intérieur du corps récepteur (11) et disposés à une certaine distance l'un de l'autre, que les deux anneaux (14, 15) sont orientés parallèlement un à l'autre et perpendiculairement à l'axe longitudinal de l'appareil (2), que dans les deux anneaux respectifs (14, 15), il est inséré une multitude de corps roulants saillant de ceux-ci, de préférence sous la forme de billes (16) portant sur l'enveloppe extérieure (13) de l'appareil (2), et que les corps roulants (16) sont logés en rotation dans l'anneau respectif (14, 15).

5. Dispositif d'après la revendication 4,
**caractérisé en ce que**
les deux anneaux (14, 15) sont arrangés au niveau des deux faces frontales du corps récepteur (11) et que la courbure des anneaux (14, 15) est concentrique par rapport à l'enveloppe extérieure (13).

6. Dispositif d'après la revendication 5,
**caractérisé en ce que**
chacun des corps roulants (16) porte perpendiculairement sur l'enveloppe extérieure (13) de l'appareil (2).

7. Dispositif d'après une ou plusieurs des revendications précédentes,
**caractérisé en ce que,**
entre le corps récepteur (11) et le bras (6), il est prévu une articulation (21) qui permet d'aligner l'appareil (2) perpendiculairement à l'axe (10) formé par le bras (6).

8. Dispositif d'après la revendication 7,
**caractérisé en ce que**
l'articulation (21) consiste de deux éléments de guidage (22, 23) tournant l'un dans l'autre et liés ensemble sous la forme d'un guidage en queue d'aronde (22).

9. Dispositif d'après la revendication 8,
**caractérisé en ce que**
les ouvertures dans le guidage en queue d'aronde (22) sont obturées en direction de l'axe longitudinal (10) du bras (6) par un couvercle (23) ou un objet similaire.

10. Dispositif d'après une ou plusieurs des revendications précédentes,
**caractérisé en ce que,**
à l'intérieur du corps récepteur (11), il est prévu un frein (17) qui se laisse actionner de l'extérieur, qui porte sur l'enveloppe extérieure (13) et qui agit de manière réglable sur celle-ci.

11. Dispositif d'après la revendication 10,
**caractérisé en ce que**
le frein (17) consiste d'un anneau divisé enveloppant entièrement l'enveloppe extérieure (13) et dont les deux extrémités libres se laissent écarter de l'extérieur moyennant un élément de réglage (18), et que les deux extrémités libres du frein (17) sont pressées l'une contre l'autre moyennant un ressort de rappel (19).

12. Dispositif d'après les revendications 10 ou 11,
**caractérisé en ce que**
le frein (17) est arrangé dans le plan central du corps récepteur (11).

13. Dispositif d'après une ou plusieurs des revendications précédentes,
**caractérisé en ce que**
le bras (6) est formé par deux jambes d'appui (8) glissant da manière télescopique l'une dans l'autre.

14. Dispositif d'après une des revendications précédentes,
**caractérisé en ce que**
pour l'alignement manuel de l'appareil (2), il est prévu sur celui-ci une ou plusieurs poignées (25).

15. Dispositif d'après une des revendications précédentes,
**caractérisé en ce que**
la surface de l'appareil (2) orientée parallèlement à son axe longitudinal (9) au niveau de l'enveloppe extérieure (13), sert de butée (28) pour la liberté de mouvement de l'appareil (2).

16. Dispositif d'après une des revendications 1 ou 2,
**caractérisé en ce que**
l'angle de réglage (β) est subdivisé en deux plages de réglage différentes.
